# EUROPÄISCHE PATENTSCHRIFT

(11) **EP 0 689 874 B1**
(45) Veröffentlichungstag und Bekanntmachung des Hinweises auf die Patenterteilung: **26.09.2001**
(21) Anmeldenummer: 95108994.5
(22) Anmeldetag: 12.06.1995
(51) Int. Cl.: B05B 7/04, B05B 7/10, B05B 11/00, B05B 11/02, B05C 17/005, A61B 17/00

(54) **Vorrichtung zum Versprühen eines Gemischs aus zwei Komponenten**
Apparatus for spraying a mixture of two components
Appareil de pulvérisation d'un mélange à deux composants

(30) Priorität: 28.06.1994 DE 4422505
(43) Veröffentlichungstag der Anmeldung: 03.01.1996
(73) Patentinhaber: Aventis Behring Gesellschaft mit beschränkter Haftung, 35002 Marburg (DE)
(72) Erfinder: Reidel, Guillermo, Dr., D-35043 Marburg (DE); Weitzel, Dietmar, Dr., D-35043 Marburg (DE); Grothoff, Hans, Dr., D-44225 Dortmund (DE)

(56) Entgegenhaltungen:
- EP-A- 0 037 393
- EP-A- 0 210 160
- EP-A- 0 479 451
- EP-A- 0 526 824
- GB-A- 1 067 717
- US-A- 4 978 336
- US-A- 4 979 942

## Beschreibung

Die Erfindung betrifft eine Vorrichtung enthaltend zwei Spritzen, deren Mündungen in einem Sprühkopf mit Sprühdüse (Zerstäuber) enden. Diese Vorrichtung ist geeignet, zwei Flüssigkeiten als Gemisch zu versprühen und gegebenenfalls auf eine Fläche aufzubringen. Vorzugsweise sind diese Flüssigkeiten die Komponenten eines Klebersystems, besonders eines Gewebeklebers.

Zweikomponenten-Gewebekleber, die auf der Reaktion von Fibrinogen mit Thrombin unter Bildung eines Fibringerinnsels beruhen, werden bei großflächiger Anwendung üblicherweise versprüht, um gleichmäßige, dünne Fibrinschichten zu erhalten. Bekannt dafür ist eine Vorrichtung (EP-A-0 302 411, im folgenden als (1) bezeichnet), wobei die mit den Komponenten gefüllten Applikationsspritzen auf einen Sprühkopf gesteckt wird. Dieser Sprühkopf weist zwei getrennte Kanäle für beide Komponenten auf, und besitzt einen Luer-Lock-bestückten Anschluß für Druckluft, wodurch die Komponenten durch einfaches gleichzeitiges vorwärtsdrücken der beiden Spritzenkolben versprüht werden können.
Trotz der guten Sprühergebnisse, die mit diesem System erzielt werden können, ist dieses mit erheblichen Anwendungsnachteilen verbunden, die einen breiteren Einsatz verhindert haben:
- es benötigt einen Druckluft- oder Treibgasanschluß, der in den meisten deutschen Operationssälen genormt vorhanden ist. Außerhalb Deutschlands allerdings haben sich andere inkompatible Normen durchgesetzt, oder die Anschlüsse fehlen völlig;
- es benötigt einen Druckminderer bzw. -regler, um den hohen Druck einer zen tralen Druckluftversorgung auf einen Arbeitsdruck von ca. 1,5 bis 2,5 bar zu re duzieren;
- die aus dem Druckluftanschluß austretende Pressluft ist naturgemäß unsteril, und muß daher mittels eines Spezialfilters steril gefiltert werden;
- der Verbindungsschlauch zwischen Wandanschluß und dem Sprühsystem muß lang genug sein, um universell eingesetzt werden zu können. Aus diesem Grund werden sie üblicherweise eher zu lang angeboten, so daß sie als Stolperfallen für das OP-Personal angesehen werden können.
- die Zerstäubung außerhalb der Reichweite des Druckschlauches ist nicht möglich, wodurch das System nicht im Rettungswagen, im ambulanten Einsatz oder im niedergelassenen Bereich eingesetzt werden kann; und
- der Zusammenbau dieses Systems ist zeitaufwendig und bietet verschiedene Fehlerquellen, die sich im Klinikalltag als störend, ja sogar als gefährlich erweisen können.

Eine ähnliche Vorrichtung wie in (1) ist auch in EP-A-0 037 393 (2) beschrieben.

In US 4,978,336 ist eine Vorrichtung zum Versprühen von 2 Flüssigkeiten als Gemisch beschrieben, wobei diese Vorrichtung zwei Spritzen zur Aufnahme der Flüssigkeiten enthält. Das Totvolumen im Sprühkopf wird dabei durch einen Einsteckkörper verringert. Aufgabe dieser Erfindung war es, ein System zur Verfügung zu stellen, das diese Nachteile nicht besitzt.

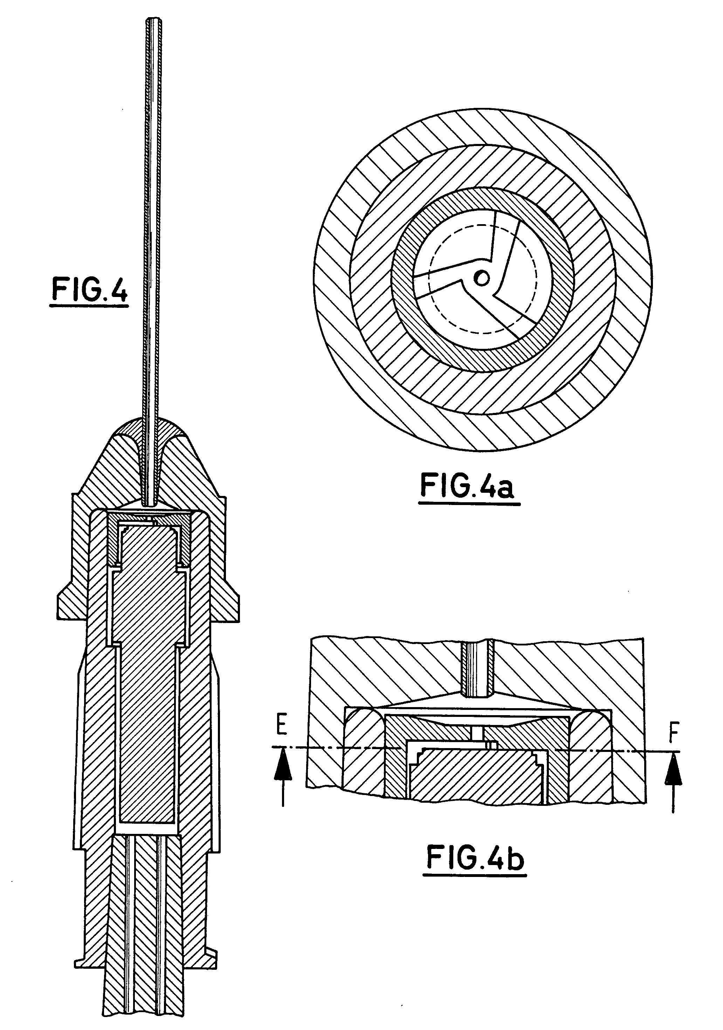

Aus beiden genannten Veröffentlichungen (1) und (2) geht die Notwendigkeit zur Durchmischung der beiden Komponenten als ein wesentliches Indiz hervor. Die Nachteile von (2) in dieser Richtung sind bereits in (1) hinreichend dargestellt. Aber auch (1) weist, neben dem generellen Nachteil der Preßluftabhängigkeit, in diesem Punkt noch erhebliche Nachteile auf. So ist der von (1) gegenüber (2) hervorgehobene Mangel der unzureichenden Durchmischung der Komponenten auch mit (1) nicht beseitigt. Zwar hat (1) das Problem der Entfernungabhängigkeit (Schnittpunktausrichtung) von (2) offensichtlich verringert, trotzdem aber letztlich keine wesentliche Verbesserung hinsichtlich der Mischungsgenauigkeit und des Homogenisierungsgrades bewirkt. Stattdessen ist festzustellen, daß das Mischungsverhältnis der Komponenten zueinander, gemessen über die Gesamtfläche des erzeugten Sprays, außerordentlich stark variiert. Insbesondere in den Randbereichen der besprühten Fläche sind die Einzelkomponenten in unvermischter Form anzutreffen, wie dies auch aus Fig. 4 (1) abzuleiten ist. Je weiter die Komponentenanteile vom Trennsteg entfernt sind, desto weniger intensiv werden sie in den Mischvorgang miteinbezogen. Dieser negative Effekt verstärkt sich bei geringen Sprühdistanzen noch uind ist weiterhin abhängig vom Preßluftdruck. Das erhaltene Resultat befriedigt somit in der Praxis keineswegs. Neben der umständlichen Handhabung durch die zusätzliche Preßluftversorgung sind durch die Inhomogenität der Mischung sowohl die Festigkeits- und Haftungswerte der erzeugten Verklebungen bzw. Versiegelungen als auch die Oberflächeneigenschaften der Filme nicht optimal.

Die Erfindung vermeidet die vorgeschilderten Nachteile und Schwierigkeiten und stellt sich zur Aufgabe, eine Vorrichtung zu schaffen, die sicherstellt, daß bereits vor der eigentlichen Ausbringung der Komponenten innerhalb der Vorrichtung sowohl das vorgeschriebene Mischungsverhältnis der Komponenten zueinander exakt eingestellt wird als auch eine gleichmäßige Vermischung der Komponenten miteinander erfolgt, wobei dieser Vorgang als auch die eigentliche Applikation rein manuell ohne zusätzliche Hilfsenergie durchführbar sein sollte, womit eine Beeinflussung durch äußere Faktoren oder durch Manipulationen weitestgehend auszuschließen ist.

Die Aufgabe wird erfindungsgemäß dadurch gelöst, daß Einzelkomponenten über zwei oder mehrere, miteinander bewegungs- und ausgangsgekoppelte handelsübliche Spritzenkörper in eine Vormischkammer dosiert werden, von dort nach dem Durchlaufen einer Vormischstrecke über mehrere Querkanäle in eine Homogenisierkammer gelangen, wo eine endgültige Vermischung der Einzelströme und Homogenisierung der Einzelkomponenten erfolgt und die eigentliche Applikation erst nach Verlassen der Homogenisierungskammer stattfindet.

Zerstäuber zum manuellen Versprühen von Kosmetika, Reinigungsmittel und andere Flüssigkeiten ohne Verwendung von Druck- oder Treibgas sind bekannt.

Das Versprühen von Zweikomponenten-Gewebeklebern ohne Verwendung von Druck- oder Treibgas, mit einer Durchmischung und damit verbunden mit einem Start der Polymerisationsreaktion der Komponenten noch vor Verlassen des Sprühkörpers, galt bisher als nicht möglich. Zum einen handelt es sich bei den Komponenten, insbesondere beim Fibrinogenkonzentrat, um hochvisköse Proteinlösungen, und zum anderen reagieren die Komponenten bei Durchmischung heftig miteinander, wobei die Gerinnung innerhalb weniger Sekunden (ca. 4-5 Sek.) einsetzt, so daß eine Verstopfung der Sprühdüse befürchtet werden mußte.

In den USA ist ein Thrombinpräparat bekannt, das für die lokale Blutstillung mittels eines einfachen beigepackten sterilen Zerstäubers versprüht werden kann. Dieses ist bei Thrombinlösungen komplikationslos möglich, weil sie als inerte, dünnflüssige Lösung zum Einsatz kommen, so daß die Sprühdüse nicht verstopfen kann.

Dieser Zerstäuber ist jedoch für Gewebekleber ungeeignet. Der Klebstoff kann nicht versprüht werden, da die Sprühdüse innerhalb kurzer Zeit verstopft wird.

Es konnte ein Zerstäuber (Sprühkopf mit Sprühdüse) entwickelt werden, bei dem das Totvolumen und damit der Verlust an wertvoller Substanz auf ein Minimum reduziert werden konnte. Dadurch konnte die Verweildauer der vermischten Komponenten im Zerstäuber derart verkürzt werden, daß unter Normalbedingungen kein Verstopfen der Sprühdüse beobachtet wird. Dieses System vermeidet die Nachteile der stationären Systeme.

Zudem ist der erfindungsgemäße Sprühkopf mit Sprühdüse leicht auszuwechseln, wenn bei der Applikation längere Zeit unterbrochen wird und ein erneutes Sprühen nicht mehr möglich ist.

Die Erfindung reduziert außerdem den anfallenden Plastikmüll erheblich.

### Arbeitsweise der Vorrichtung am Beispiel der bevorzugten Ausführungsform:

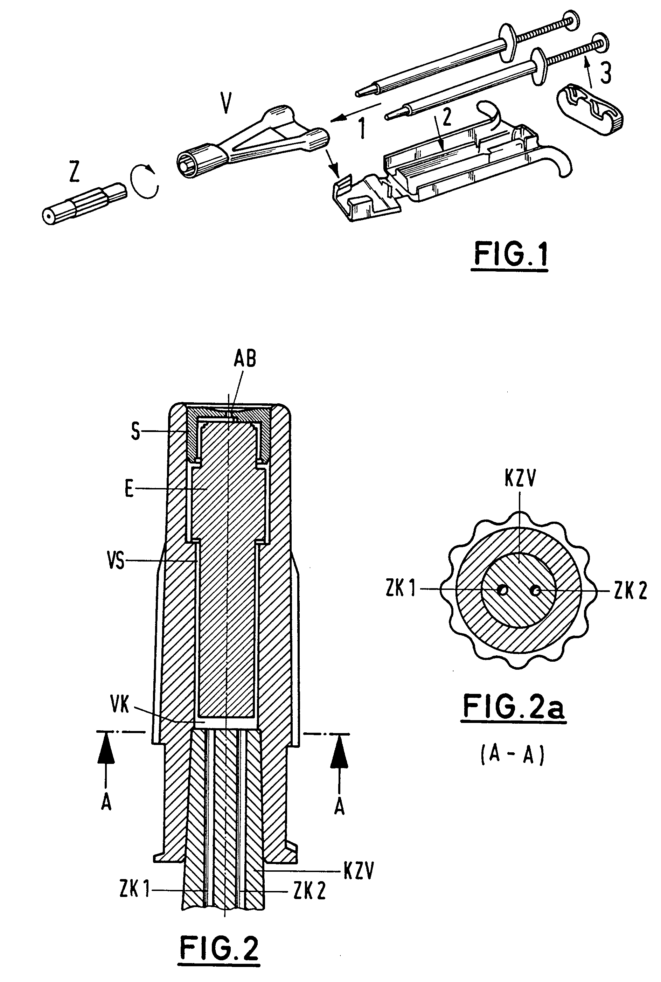

Gegenstand der Erfindung ist eine Vorrichtung zum Versprühen von zwei Flüssigkeiten als Gemisch, wobei diese Vorrichtung zwei Spritzen zur Aufnahme der Flüssigkeiten enthält, deren Mündungen mit einem Verbindungsstück verbunden sind, das die beiden Flüssigkeiten zusammenführt und in einem Sprühkopf mit Sprühdüse endet. Die beiden Flüssigkeiten sind vorzugsweise die Komponenten eines Zweikomponentenklebers, besonders eines Gewebeklebers auf Fibrinogenbasis. Eine bevorzugte Ausführungsform ist in den Figuren 1, 2 und 3 dargestellt. Gegenstand der Erfindung sind jedoch selbstverständlich auch äquivalente Ausführungsformen.

Für Gewebeklebungen mittels Zweikomponenten-Klebern werden üblicherweise erst die 2 Komponenten zubereitet, indem die in fester Form vorliegenden Komponenten im jeweiligen Lösungsmittel aufgelöst und kurz vor Anwendung in die Applikationsspritzen aufgezogen werden.

Die so erhaltenen, mit Fibrinogen- und Thrombinlösung gefüllten Spritzen werden in die dafür vorgesehenen Öffnungen des Y-förmigen Verbindungsstücks gesteckt (siehe Fig. 1, Schritt Nr.1) und in die konische Aussparung der Spritzenhalterung eingelegt. Die Spritzen werden dann in die Spritzenhalterung eingerastet (Schritt Nr.2). Nun werden die Spritzenstempel mit der Griffplatte verbunden (Schritt Nr.3), so daß ein Verkanten der Spritzenstempel vermieden wird.

Auf dieses System wird zuletzt der z.B. mit Luer-Lock-Anschluß versehene Zerstäuber (Sprühkopf mit Sprühdüse) aufgeschraubt bzw. aufgesteckt (Schritt Nr.4).

Zum Versprühen wird aus einer Entfernung von etwa 10 bis 20 cm zum Sprühobjekt mit einem zügigen, gleichmäßigen Daumendruck auf die Griffplatte der Inhalt der Applikationsspritzen durch beide Arbeitskanäle des Y-förmigen Verbindungsstücks gedrückt, in der Vormischkammer des Zerstäubers (Sprühkopf mit Sprühdüse) vermischt, und durch die durch Einsteckkörper (E) verursachte Querschnittsverengung des lichten Raumes unter wachsender Geschwindigkeit bis in die äußere Sprühdüse (S) getrieben. Der Gewebekleber verläßt die Sprühdüse als feiner, gleichmäßiger Nebel und bedeckt das Gewebe als feiner Film.

Falls durch eine Unterbrechung der Applikation der Zerstäuber (Sprühkopf mit Sprühdüse) verstopft, kann er leicht durch einen neuen ersetzt werden.

In einer weiteren vorteilhaften Anwendung wird der Sprühkopf mit Sprühdüse (Zerstäuber) als Homogenisiervorrichtung genutzt für eine Applikation mittels auf die Sprühdüse aufgesteckter Nadel. Dadurch kann der Gewebekleber zielgerichtet auf kleine Klebestellen aufgebracht werden, wobei die Klebewirkung aufgrund der guten Durchmischung sehr schnell eintritt.

Dies ist anwendungstechnisch von Vorteil, da die Vorrichtung dann schon mit bereits aufmontiertem Sprühkopf enthaltend die Sprühdüse geliefert werden kann und nach Einsetzen der gefüllten Spritzenkörper für die Sprühanwendung ohne weitere Montage sofort einsetzbar ist. Für den Fall der Nadelapplikation wird diese dann lediglich zusätzlich aufgesteckt. Da dieser Wechselvorgang sehr schnell vorgenommen werden kann, kann während einer Anwendung von einer auf die andere Applikationsform umgeschaltet werden, ohne daß zu befürchten ist, daß die Komponenten für die weitere Benutzung zuweit ausgehärtet sind und die Spritzenkolben blockieren, weil die Sprühdüse verstopft ist.

Weitere Einzelheiten zeigen Fig. 1, 2 und 3, insbesondere die vorteilhaften Wirkungen des Einsteckkörpers:
- Reduzierung des Totvolumens
- Begrenzung der Düseneinstecktiefe
- Vormischfunktion durch mehrfache Umlenkung des Volumenstroms
- Abdeckung der Homogenisierkammer und Teilüberdeckung der Querkanäle.

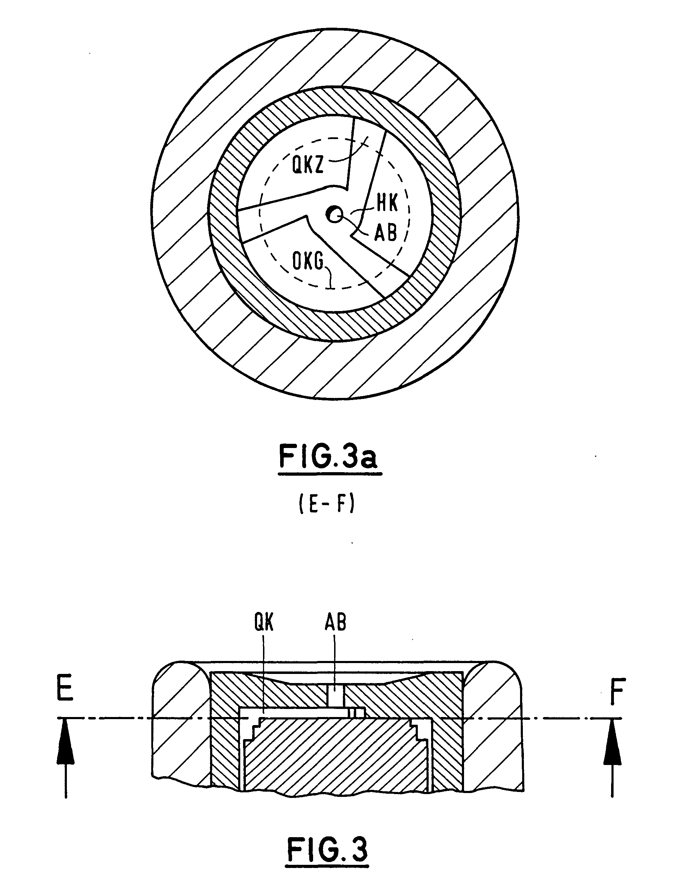

Zu
- **Fig. 1** (Zerstäuber): Einbau der Sprühkopfes mit Sprühdüse in die Zweikomponentenklebervorrichtung
Z Zerstäuber (Sprühkopf mit Sprühdüse)
V Verbindungsstück
- **Fig. 2**: Längsschnitt durch den Sprühkopf mit Sprühdüse
AB Austrittsbohrung
S Sprühdüse
E Einsteckkörper
VS Vormischstrecke
VK Vormischkammer
ZK1 Zufuhr Komponente 1
ZK2 Zufuhr Komponente 2
KZV Kupplungszapfen Verbindungsstück
- **Fig. 2a**: Querschnitt A - A
- **Fig. 3a**: Querschnitt durch den Sprühdüsenteil
QKZ Querkanal-Zutritt
QKG Querkanal-Überdeckungsgrenze
HK Homogenisierkammer
AB Austrittsbohrung
- **Fig. 3**: Längsschnitt durch den Sprühdüsenteil
QK Querkanal
- **Fig. 4**: Zerstäuber mit aufgesteckter Spritzennadel
- **Fig. 4a**: Querschnitt durch.den Sprühdüsenteil
QKZ Querkanal-Zutritt
QKG Querkanal-Überdeckungsgrenze
HK Homogenisierkammer
AB Austrittsbohrung
- **Fig. 4b**: Längsschnitt durch den Sprühdüsenteil
QK Querkanal

## Patentansprüche

1. Vorrichtung zum Versprühen eines Gemischs einer ersten und zweiten Flüssigkeit enthaltend
(i) eine erste und zweite Spritze zur Aufnahme und mit Auslaß für die erste und zweite Flüssigkeit,
(ii) ein Verbindungsstück mit ersten und zweiten Kanälen zur Zusammenführung des ersten und zweiten Spritzenauslaßes, wobei die genannten Kanäle in einer Vormischkammer zum Vormischen der ersten und zweiten Flüssigkeit zwecks Herstellung einer Mischung enden,
(iii) daran anschließend einen Bereich mit verringertem Totvolumen, wobei dieser Bereich mit verringertem Totvolumen in eine Homogenisierkammer zur Homogenisierung der Flüssigkeit mündet, und
(iv) eine Austrittsöffnung zum Versprühen der Flüssigmischung.

2. Vorrichtung nach Anspruch 1, **dadurch gekennzeichnet, daß** sie eine Halterung zur Aufnahme der Spritzen besitzt.

3. Vorrichtung nach Anspruch 1, **dadurch gekennzeichnet, daß** die Spritzenstempel mit einer Griffplatte verbunden sind.

4. Vorrichtung gemäß Anspruch 1 bis 3, **dadurch gekennzeichnet, daß** die genannte Vormischkammer, der Bereich mit verringertem Totvolumen und die Homogenisierkammer in einem Sprühkopf ausgebildet sind, der leicht auswechselbar ist.

5. Vorrichtung nach Anspruch 1 bis 4, **dadurch gekennzeichnet, daß** der genannte Bereich mit verringertem Totvolumen und die genannte Homogenisierkammer durch eine Vielzahl von Querkanälen getrennt sind.

6. Vorrichtung nach Anspruch 1 bis 5, **dadurch gekennzeichnet, daß** die Spritzen mit den Komponenten eines Mehrkomponentenklebers gefüllt sind.

7. Vorrichtung nach Anspruch 6, **dadurch gekennzeichnet, daß** der Mehrkomponentenkleber ein Gewebekleber ist.

8. Verwendung einer Vorrichtung nach Anspruch 1 bis 5, **dadurch gekennzeichnet, daß** sie zum Aufbringen eines Mehrkomponentenklebers gebraucht wird.

9. Verwendung nach Anspruch 8, **dadurch gekennzeichnet, daß** ein Gewebekleber versprüht wird.

## Claims

1. An apparatus for spraying a mixture of a first and second liquid comprising
(i) a first and second syringe to receive and with outlet for the first and second liquid,
(ii) a connecting piece with first and second channels to bring the first and second syringe outlet together, where said channels terminate in a premixing chamber for premixing the first and second liquid to produce a mixture,
(iii) subsequent thereto a region with reduced dead volume, where this region with reduced dead volume opens into a homogenization chamber for homogenizing the liquid, and
(iv) an outlet orifice for spraying the liquid mixture.

2. An apparatus as claimed in claim 1, which has a holder to receive the syringes.

3. An apparatus as claimed in claim 1, wherein the syringe plungers are connected to a gripping plate.

4. An apparatus as claimed in any of claims 1 to 3, wherein the said premixing chamber, the region with reduced dead volume and the homogenization chamber are formed in a spray head which is easily exchangeable.

5. An apparatus as claimed in any of claims 1 to 4, wherein the said region with reduced dead volume and the said homogenization chamber are separated by a plurality of transverse channels.

6. An apparatus as claimed in any of claims 1 to 5, wherein the syringes are filled with the components of a multicomponent adhesive.

7. An apparatus as claimed in claim 6, wherein the multicomponent adhesive is a tissue adhesive.

8. The use of an apparatus as claimed in any of claims 1 to 5, wherein it is utilized to apply a multicomponent adhesive.

9. The use as claimed in claim 8, wherein a tissue adhesive is sprayed.

## Revendications

1. Appareil de pulvérisation d'un mélange d'un premier et un second liquides, contenant :
(i) un premier et un second injecteurs pour recevoir et évacuer le premier et le second liquides,
(ii) un raccord avec un premier et un second canaux pour combiner la première et la deuxième sorties des injecteurs, les canaux mentionnés se terminant par une chambre de pré-mélange pour pré-mélanger le premier et le second liquides afin de réaliser un mélange,
(iii) s'y raccordant une zone avec un volume mort réduit, cette zone à volume mort réduit débouchant dans une chambre d'homogénéisation pour homogénéiser le liquide, et
(iv) une ouverture de sortie pour pulvériser le mélange de liquides.

2. Appareil selon la revendication 1, **caractérisé en ce qu'**il présente un support pour la réception des injecteurs.

3. Appareil selon la revendication 1, **caractérisé en ce que** les pistons d'injection sont reliés à une plaque de préhension.

4. Appareil selon les revendications 1 à 3, **caractérisé en ce que** la chambre de pré-mélange mentionnée, la zone à volume mort réduit et la chambre d'homogénéisation sont formées dans une tête de pulvérisation, qui peut être facilement remplacée.

5. Appareil selon les revendications 1 à 4, **caractérisé en ce que** la zone mentionnée avec un volume mort réduit et la chambre d'homogénéisation mentionnée sont séparées par une pluralité de canaux transversaux.

6. Appareil selon les revendications 1 à 5, **caractérisé en ce que** les injecteurs sont remplis des composants d'une colle à plusieurs composants.

7. Appareil selon la revendication 6, **caractérisé en ce que** la colle à composants multiples est une colle à tissus.

8. Utilisation d'un appareil selon les revendications 1 à 5, **caractérisée en ce qu'**il est utilisé pour appliquer une colle à plusieurs composants.

9. Utilisation selon la revendication 8, **caractérisée en ce qu'**on pulvérise une colle à tissus.
